# EUROPEAN PATENT APPLICATION

(11) **EP 2 292 260 A1**
(43) Date of publication of application: **09.03.2011**
(21) Application number: 09290627.0
(22) Date of filing: 13.08.2009
(51) Int. Cl.: A61K 39/04

(54) **Use of mycobacterium bovis BCG killed by extended freeze drying (EFD) for preventing or treating atherosclerosis**

(71) Applicant: INSTITUT PASTEUR, 75015 Paris (FR); INSTITUT NATIONAL DE LA RECHERCHE AGRONOMIQUE, 75007 Paris (FR)
(72) Inventor: Marchal, Gilles, 75014 Paris (FR); Lagranderie, Micheline, 92200 Neuilly Sur Seine (FR); Schwartz-Cornil, Isabelle, 78350 Jouy en Josas (FR)
(74) Representative: Thomas, Dean

(57) **Abstract**

Use of *Mycobacterium bovis* BCG killed by Extended Freeze Drying (EFD) for preventing or treating atherosclerosis.

## Description

The invention relates to the use of *Mycobacterium bovis* BCG killed by Extended Freeze Drying (EFD) for preventing or treating atherosclerosis.

Atherosclerosis is a complex chronic inflammatory process that progresses over decades. It is characterized by the accumulation of oxidized low-density lipoproteins (LDL), increased cell death and hypertrophic degeneration of the arterial wall, causing narrowing of the channel and thus impairing blood flow. It can occur in any area of the body, but is most important when it happens in the heart, brain or blood vessels leading to the brain. The narrowing is due to the formation of plaques (raised patches) in the inner lining of the arteries. These plaques consist of low-density lipoproteins, decaying muscle cells, fibrous tissue, clumps of blood platelets, cholesterol, macrophages, T-lymphocytes and sometimes calcium. They tend to form in regions of turbulent blood flow and are found most often in people with high concentrations of cholesterol in the bloodstream. The number and thickness of plaques increases with age, causing loss of the smooth lining of the blood vessels and encouraging the formation of thrombi (blood clots). Sometimes fragments of thrombi break off and form emboli, which travel through the bloodstream and block smaller vessels.

Atherosclerosis and its clinical manifestations are a major cause of morbidity and mortality among both men and women. Atherosclerotic heart disease, involving the coronary arteries (coronary heart disease), is the most common cause of death, accounting for one-third of all deaths. Atherosclerotic interference with blood supply to the brain (stroke) is the third most common cause of death after cancer. Vascular insufficiency is another clinical manifestation of atherosclerosis which causes a great deal of serious illness by reducing the flow of blood in other major arteries, such as to the kidneys, legs, and intestines.

Unfortunately, atherosclerosis produces no symptoms until the damage to the arteries is severe enough to restrict blood flow. Restriction of blood flow to the heart muscle due to atherosclerosis can cause angina pectoris or a myocardial infarction (a heart attack). Restriction of blood flow to the muscles of the legs causes intermittent claudication (pains in the legs brought about by walking and relieved by rest). Narrowing of the arteries supplying blood to the brain may cause transient ischemic attacks (symptoms and signs of a stroke lasting less than 24 hours) and episodes of dizziness, or ultimately, to a stroke itself.

Current medical treatment of atherosclerosis includes the use of several drugs such as Statins (rosuvastatin) and fibrates. Besides anticoagulant drugs used to try to minimize secondary clotting and embolus formation, vasodilator drugs provide symptom relief but are of no curative value. Surgical treatment is available for those unresponsive to medical treatment or in certain high-risk situations. Balloon angioplasty can open up narrowed vessels and promote an improved blood supply. The blood supply to the heart can also be restored by coronary artery bypass surgery. Large atheromatous and calcified arterial obstruction can be removed by endarterectomy, and entire segments of diseased peripheral vessels can be replaced by woven plastic tube grafts.

The pathogenesis of atherosclerosis involves a complex interplay of inflammation, autoimmunity and tissue-specific degeneration.

Multiple risk factors for atherosclerosis include hypertension (high blood pressure), smoking, diabetes mellitus, dyslipidaemia (hypercholesterolemia), metabolic syndrome, obesity, ischemia, male gender, age, family history of heart disease, and a sedentary lifestyle.

Whatever the factors, the resulting chronic inflammation of the arterial intima is the principal driving force behind the development of atherosclerosis. The major cellular processes involved in atherosclerosis include activated endothelial cells which release chemokines Oxidized low-density lipoproteins (LDL) induce activated endothelial cells to express glycoprotein adhesion molecules (vascular cell adhesion molecule-1 (VCAM-1), intracellular cell adhesion molecule-1 (ICAM-1) and E-selectin) that aid in the recruitment and infiltration of inflammatory cells. After binding to the endothelium, leucocytes infiltrate into the intima. There, monocytes differentiate into macrophages, which sequester cholesterol from oxidized LDL and thereby become foam cells. The resulting accumulation of fatty streaks upon the arterial wall is followed by the formation of advanced atherosclerotic lesions. Vascular smooth muscle cell proliferate and migrate toward the lesion. Upon their recruitment, vascular smooth muscle cells secrete extracellular matrix that forms a fibrous plaque over the lipid atheroma. Ultimately, unstable plaques may rupture, as a result of ongoing chronic inflammation. The resulting thrombi are responsible for acute coronary events such as myocardial infarction and stroke.

Activation of the transcription factors NF-κB has been linked to the onset of atherosclerosis. The phosphorylation of I-κB activates NF-κB playing a role in this disease process. However, there is no direct evidence that NF-κB activation is necessary for atherosclerosis (T. Collins and M.I Cybulsky, J. Clinical Investigation, 2001, 107, 255-262).

Targeting the inflammation represents an approach for developing treatments for atherosclerosis. Some anti-inflammatory drugs were shown to exert cardioprotective effects at key stages of atherogenesis. Thiazolinediones are peroxisome proliferator-activated receptor (PPAR)γ agonists that are in clinical use for the treatment of type 2 diabetes. PPARγ regulates numerous cellular processes contributing to atherosclerosis. *In vitro* animal model and clinical studies indicate that thiazolinediones correct endothelial dysfunction, suppress chronic inflammatory processes, reduce fatty streak formation, delay plaque evolution and vessel wall thickening and enhance plaque stabilization. Thus thiazolinediones show potential as potent anti-inflammatory, antithrombic agents that could both improve glucose levels and the long-term cardiovascular risk related to atherosclerosis in patients with type 2 diabetes (B. Staels, Current Medical Research and Opinion, 2005, 21, suppl 1, S13-S20).

More recently, an increasing body of evidence suggests that the immune system is a major factor modulating the atherogenic process (for a review see Nilsson, J. and G.K. Hanson, Journal of Internal Medecine, 2008, 263, 464-478). The disease process is associated with local formation of modified self antigens (oxidized LDL, HSP, apoptotic fragments) that are targeted by both innate and adaptative immune responses. It is likely that these autoimmune responses have a beneficial effect facilitating the removal of potentially harmful rest products from oxidized LDL and dying cells. However, if the balance between protective and disease-promoting autoimmunity is lost, the autoimmune system may accelerate into a destructive pro-inflammatory process causing plaque growth and destabilization. These observations point to the possibility of developing new treatments for atherosclerosis based on modulation of immune responses against plaque antigens.

Animal studies have provided proof-of-principle support that it is possible to inhibit the development of atherosclerosis by modulating immune responses against plaque antigens by vaccines based on antigens present in oxidized LDL. However, as oxidized LDL is a complex particle with an antigen composition that it is difficult to standardize and as it potentially may also contain harmful antigens it is in itself not an ideal vaccine component. Over the last few years considerable effort has therefore been put on characterizing the exact antigens in oxidized LDL that induce athero-protective immunity. Two antigens were identified in oxidized LDL: oxidized phospholipids (phosphorylcholine) and apo B-100 peptides. A limitation with both phosphorylcholine and apo B peptide-based vaccines, is that the mechanism of action is poorly understood. Oxidized phospholipids are recognized by a subclass of IgM referred to as natural antibodies, whose regulation of expression is poorly understood, and which cross-react with self antigens associated with senescent cells and cellular debris. The high specificity and the possibility of producing standardized vaccine preparations are some advantages of apo B peptide-based vaccines and human vaccines are presently in preclinical development. The disadvantages with this approach include the need for human leucocyte antigen (HLA) genotyping of patient before treatment and the risk that the vaccines need to be individualized depending on HLA type.

However, besides the apo B peptide-based vaccines, no other therapeutic approach targeting the immune system has been developed for the treatment of atherosclerosis, so far.

There are several ways for the body to control the activity of the autoimmune responses, an important one being the regulatory T cells (Tregs). Several subtypes of Tregs have been identified. Natural CD4⁺ CD25⁺ Foxp3⁺ Tregs are developed in the thymus and then enter peripheral tissues where they constitute 5-10 % of all T cells. In contrast to natural Tregs, the IL-10-producing T regulatory 1 (Tr1) and TGF-β-producing T helper 3 (Th3) cells are generated from naive T cells in the periphery following antigen presentation and activation by dendritic cells (DCs). Several lines of evidence have implicated dysregulation of Treg function in atherosclerosis. There is also accumulating functional evidence for a protective role of Tregs in atherosclerosis. Depletion of natural Tregs through deletion of CD80/86, CD28 or ICOS as well as anti-CD25 antibody treatment significantly increases plaque formation (Ait-Oufella et al., Nat. Med., 2006, 12, 178-180; Gotsman et al., Circulation, 2006, 114, 2047-2055). Similarly, inhibition of Th3 cells through deletion of the T-cell receptor for TGF-β markedly enhances the progression of the disease (Roberson et al., J. Clin. Invest., 2003, 112, 1342-1350) whilst administration of a clone of ovalbumin-specific Tr1 cells together with its cognate antigen inhibited plaque development in Apolipoprotein E deficient (ApoE^{-/-}) mice (Mallat et al., Circulation, 2003, 108, 1232-1237). The understanding of the role of regulatory T cells in atherosclerosis is still very incomplete.

For example, *Mycobacteria bovis* BCG killed by Extended Freeze Drying (EFD) was shown to be a potent anti-inflammatory agent in several animal models of allergy (asthma; International PCT Application WO 03/049752) and intestinal inflammatory disease (inflammatory bowel disease; International PCT Application WO 2007/072230). EFD does not cause the toxic side effects associated with live or heat-killed BCG and does not interfere with the diagnosis of tuberculosis by the DTH skin-test. EFD has the original property of acting not only on the Th1 signalization pathway to which TNF-α, IL-12, IFN-γ and T-bet are associated, but also on the Th2 signalization pathway to which IL-4, IL-13 and GATA-3 are associated and on a new signalization pathway to which IL-17 and PPAR-γ seem to be associated with (International PCT Application WO 2007/072230). The protective/curative effect of EFD against the symptoms of allergy and intestinal inflammatory disease is associated with the stimulation of CD4+ CD25+ regulatory cells producing IL-10 (International PCT Applications WO 03/049752 and WO 2007/072230).

However, they are major side effects that may be associated with the use of regulatory T cells stimulating agents for treating autoimmune diseases like atherosclerosis type of treatment (Nilsson, J. and G.K. Hanson, Journal of Internal Medecine, 2008, 263, 464-478). If regulatory T cells are too suppressive, and the immunosuppression is not restricted to the affected tissue, there is a risk that the defense against infections and the CD8 T-cell dependent tumour cell surveillance will be impaired.

By using two experimental mice models: Apolipoprotein E deficient (ApoE^{-/-}) mice which develop atherosclerosis spontaneously; and low-density lipoprotein receptor deficient (Ldlr^{-/-}) mice which develop atherosclerosis upon hyperlipidemic diet, the inventors have demonstrated that EFD can prevent atherosclerosis. Unexpectedly, the inventors have shown that the anti-inflammatory activity of EFD does not suppress the host immune defense to pathogenic microorganisms (pathogenic bacteria such as *M. tuberculosis,* parasite such as *Leishmania major* or virus such as influenza virus, for example) or the host protective immune response to vaccine antigens (BCG vaccine or *Neisseria meningitidis* vaccine, for example).

Labelling of macrophages with MOMA-2 antibodies and quantification of endothelial lesions, indicated that MOMA-2+ macrophages were less numerous in atheroma plaques and the atheroma plaques were also less extended in EFD-treated than in control mice.

The inflammatory cytokines (IL-13, KC(IL-8), MIP-1β, IL-1 β and TNFα) were decreased in the serum of all EFD-treated mice.

Some other inflammatory cytokines (IL-17, eotaxin, IL-6 and IL-12p40) were decreased in ApoE^{-/-} but not in Ldlr ^{-/-} EFD-treated mice in which their concentrations were not changed in comparison to PBS-treated mice. Based on these criteria EFD has higher efficiency in mice which develop atherosclerosis "spontaneously".

IFN-γ and IL-10 were increased after EFD treatment. IL-10 being known to be an anti-inflammatory cytokine, the large difference between PBS and EFD treatment could be a signature of EFD anti-inflammatory efficacy.

RORγt, a signature of inflammatory Th-17 cells and GATA, a signature of Th2 cells were decreased while T-bet (Th1 marker) and FOXP3, a signature of Treg cells, were enhanced after EFD treatment.

After EFD treatment, a decreased level of NFκB and a slight increase of PPARγ were also observed in nuclear extracts of spleen cells and vascular tissue.

It is noteworthy that EFD treatment increases a particular population of Treg cells expressing FOXP3 but not suppressive.

Unexpectedly, the induction of CD4+ FOXP3+ Treg cells did not impair the host immune defense to pathogenic microorganisms infection or the host protective immune responses confered by vaccines such as *Mycobacterium bovis BCG* or *Neisseria* vaccines. Moreover, EFD treatment did not modify the course of a viral infection (such as influenza). EFD does not interfere with the control of Mycobacterium tuberculosis load in infected guinea pigs. Moreover, the protective capacity of BCG vaccine is not affected, either before or after treatment with EFD. EFD treatment also does not modify the effects of Neisseria vaccination

These results indicate that EFD represents a potential new therapeutic approach for preventing atherosclerosis. EFD is expected to have a prolonged duration of activity (at least 2 months). Therefore, EFD should have the advantage of a very limited number of administration (about two to four injections per year) compared to currently used drugs which require daily repetitive administration. In addition, EFD treatment is safe since it does not sensitize to infection with pathogenic microorganisms or impair the vaccination efficiency.

The invention relates to *Mycobacterium bovis* BCG bacteria killed by extended freeze-drying (EFD) for preventing or treating atherosclerosis.

The expression "EFD", or "extended freeze-dried *Mycobacterium bovis* BCG", as used in the context of the present invention, refers in other words to a bacterial preparation which consists of killed and non-denatured *Mycobacterium bovis* BCG bacteria, and which contains less than 1.5 % of water, preferably less than 1 % of water, more preferably, less than 0.5 % of water . In the context of the present invention, non-denatured means that the structure of the *Mycobacterium bovis* BCG bacteria molecules and in particular the primary structure of the macromolecules (proteins, polysaccharides, lipids) is preserved. Preferably, the primary structure of the proteins is preserved. An example of a preserved protein in EFD is Apa, which has the same migrating characteristics in a SDS-PAGE gel as the protein extracted from living BCG (Laqueyrerie et al., Infect. Immun., 1995, 63, 4003-4010). The EFD may be prepared by extended freeze-drying or any other process which kills the bacteria while preserving the structure of its macromolecular components. Preferably, such process preserves the primary structure of the macromolecules from the bacteria cells. These processes which may be used for preparing EFD are known to those of ordinary skill in the art and include the use of physical means, such as for example, extended freeze-drying, grinding in the presence of silica or zirconium beads, use of the so-called "French-press", sonication and gamma-rays irradiation.

A fraction of EFD as described above is covered by the expression "EFD" of the invention. This fraction is selected in the group consisting of: a fraction consisting of a glycosidase-treated extract of said EFD, a fraction consisting of a DNase and/or a RNase-digested extract of said EFD, a fraction consisting of a protease-treated extract of said EFD, a fraction consisting of said EFD successively treated by a glycosidase, a DNase and/or a RNase, and finally a protease, and a fraction consisting of said EFD treated by a protease (as substilisine for example) and a DNase.

According to a preferred embodiment of the invention, the EFD is prepared according to the method as described in the International PCT Application WO 03/049752, said method comprising the steps of: (i) harvesting a culture of living bacteria cells, (ii) freezing the bacteria cells in water or in an aqueous solution of a salt, (iii) killing the frozen bacteria cells by drying them in a lyophiliser, for a time sufficient to remove at least 98.5% of the water, preferably at least 99 % of the water, more preferably at least 99.5 % of the water, and (iv) collecting the extended freeze-dried killed bacteria cells. Optionally, a step of washing of the bacteria cells in water or in an aqueous solution of a salt may be introduced between steps (i) and (ii).

According to another advantageous embodiment of the invention, said EFD is included in a pharmaceutical composition for preventing or treating atherosclerosis further comprising a pharmaceutically acceptable carrier and/or an additive and/or an immunostimulant and/or an adjuvant.

Any suitable carrier known to those of ordinary skill in the art may be employed in the pharmaceutical composition of the present invention, the type of carrier varying depending on the mode of administration. For parenteral administration, such as subcutaneous injection, the carrier preferably comprises water, saline buffer, lactose, mannitol, glutamate, a fat or a wax and the injectable pharmaceutical composition is preferably an isotonic solution (around 300-320 mosmoles). For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g. polylactic galactide) may also be employed as carriers for the pharmaceutical compositions of this invention. Suitable biodegradable microspheres are disclosed, for example in US Patent 4,897,268 and 5,075,109. The additive may be chosen among antiaggregating agents, antioxidants, dyes, flavor enhancers, or smoothing, assembling or isolating agents, and in general among any excipient conventionally used in the pharmaceutical industry. Any of the variety of adjuvants/immunostimulants may be employed in the compositions of the present invention to enhance the immune response. Most adjuvants contain a substance designed to protect the antigen from rapid catabolism or to create controlled inflammatory reactions, such as aluminium hydroxide and a non-specific stimulator of immune response. Suitable adjuvants for human administration are commercially available as, for example, aluminum hydroxide, biodegradable microspheres, monophosphoryl A and Quil A.

The pharmaceutical composition may be in a form suitable for oral administration. For example, the composition is in the form of tablets, ordinary capsules, gelatine capsules or syrup for oral administration. These gelatine capsules, ordinary capsules and tablet forms can contain excipients conventionally used in pharmaceutical formulation, such as adjuvants or binders like starches, gums and gelatine, adjuvants like calcium phosphate, disintegrating agents like cornstarch or algenic acids, a lubricant like magnesium stearate, sweeteners or flavourings. Solutions or suspensions can be prepared in aqueous or non-aqueous media by the addition of pharmacologically compatible solvents. These include glycols, polyglycols, propylene glycols, polyglycol ether, DMSO and ethanol.

In general, the composition may be administered by parenteral injection (e.g., intradermal, intramuscular, intravenous or subcutaneous), intranasally (e.g. by aspiration or nebulization), orally, sublingually, or topically, through the skin or through the rectum epithelium.

The amount of EFD present in the composition of the present invention is a therapeutically effective amount. A therapeutically effective amount of EFD is that amount necessary so that the EFD performs its role of reducing the signs of atherosclerosis without causing overly negative effects in the subject to which the composition is administered. The exact amount of EFD to be used and the composition to be administered will vary according to factors such as the species (human, animal) being treated, the type of subject (subject at risk of atherosclerosis such as dyslipidemia, metabolic syndrome, type 2 diabetes subjects), the mode of administration, the frequency of administration as well as the other ingredients in the composition.

Preferably, the composition comprises from about 10 µg to about 10 mg and more preferably from about 100 µg to about 1 mg, of EFD. By "about", it is meant that a slightly lower or higher quantity of EFD can be used.

For instance, for parenteral administration, such as subcutaneous injection, the individual to be treated could be subjected to a 1 dose schedule of from about 10 µg to about 10 mg and more preferably from about 100 µg to about 1 mg, for example 100 µg of EFD, every 3 to 6 months (2 to 4 subcutaneous injections of 100 µg of EFD per year).

The composition may be used either for preventing atherosclerosis in patients previously diagnosed of atherosclerosis, patients at high risk of arthrosclerosis or patients presenting xathoma and in particular patients presenting xanthelasma or for curing atherosclerosis in patients at the beginning of an episode of artherosclerosis.

The invention also relates to products containing EFD as defined in the invention or fractions thereof and a second product which is different from the first one, said second product being selected from the group consisting of anti-inflammatory and immunomodulatory drugs (acetylsalicylic acid or thiazolidinediones (glitazone), for example), as a combined preparation for simultaneous, separate or sequential use in the prevention and/or the treatment of artherosclerosis.

The invention relates also to a method for preventing or treating atherosclerosis, comprising the administration of an effective amount of the composition comprising EFD to a patient suffering from or susceptible to atherosclerosis.

The present invention will be further illustrated by the additional description and drawings which follow, which refer to examples illustrating the prevention of atherosclerosis by EFD treatment in two experimental models of atherosclerosis in mice. It should be understood however that these examples are given only by way of illustration of the invention and do not constitute in anyway a limitation thereof.

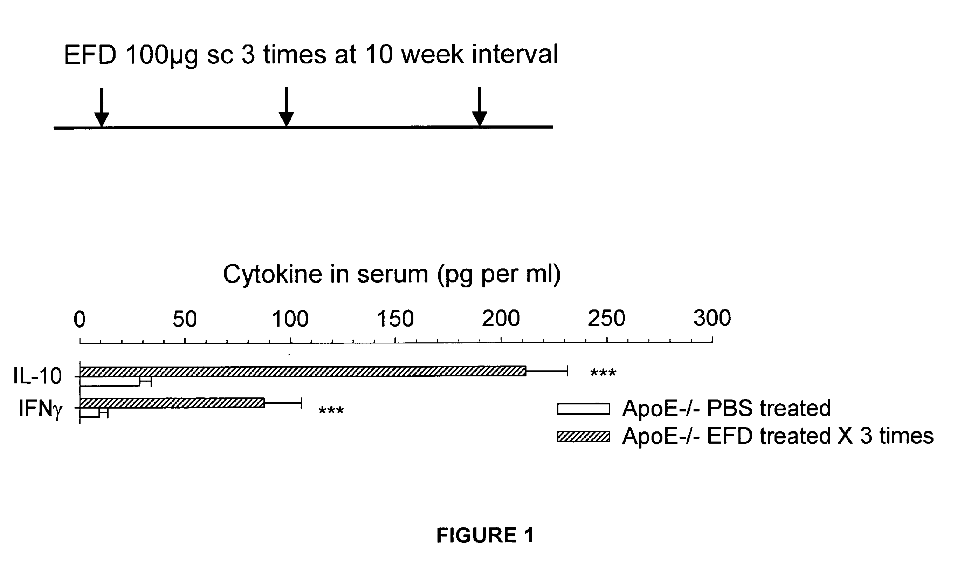

- figure 1 illustrates cytokines increased in the sera of ApoE^{-/-} mice bimonthly treated 3 times with 100 µg of EFD injected subcutaneously (SC) as compared to PBS-treated mice.

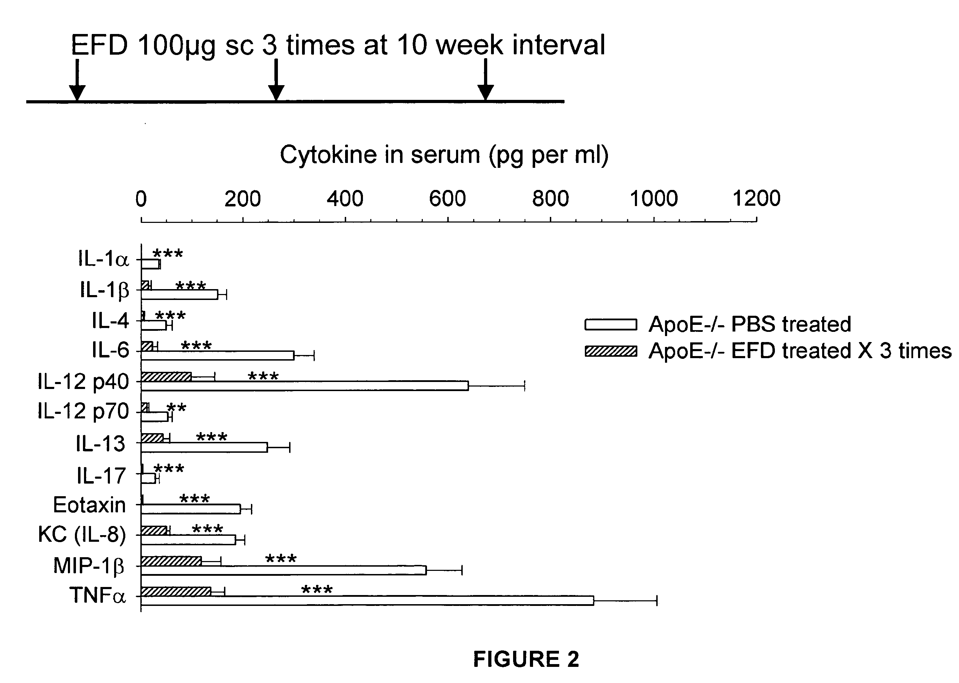

- figure 2 illustrates cytokines and chemokines reduced in the sera of ApoE^{-/-} mice bimonthly treated 3 times with 100 µg of EFD injected subcutaneously (SC) as compared to PBS-treated mice.

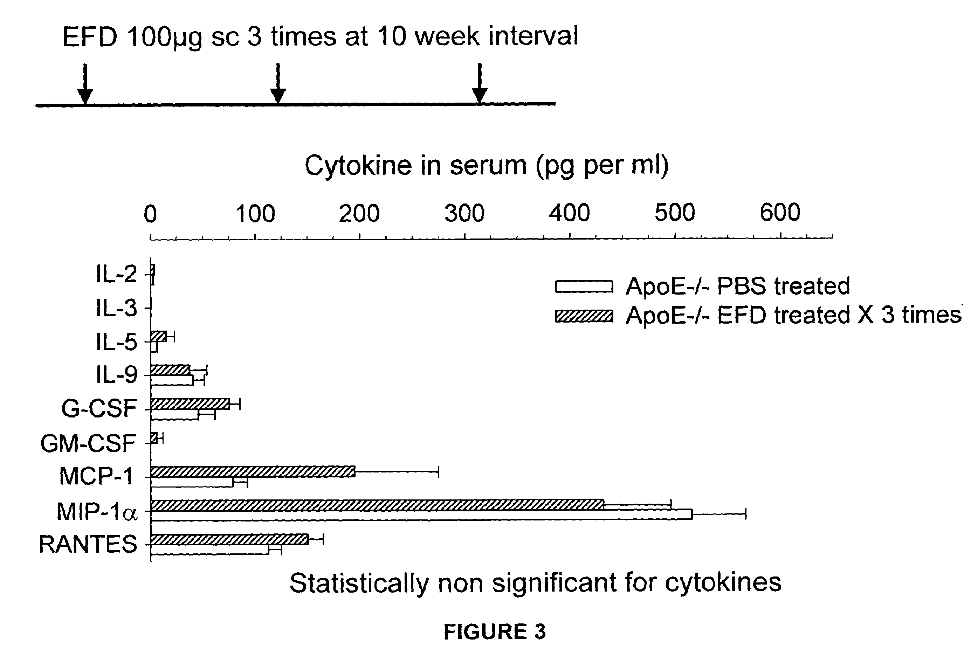

- figure 3 illustrates cytokines and chemokines remaining unchanged in the sera of ApoE-/- mice bimonthly treated 3 times with 100 µg of EFD injected subcutaneously (SC) as compared to PBS-treated mice.

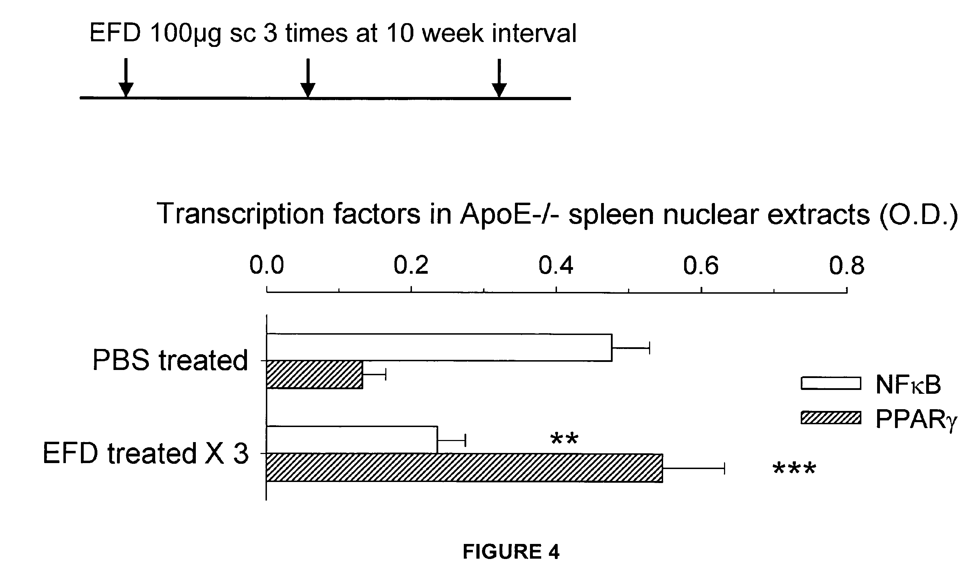

- figure 4 illustrates the decreased NFκB activation and enhanced PPARγ expression in spleen nuclear extracts of ApoE-/- mice bimonthly treated 3 times with 100 µg of EFD injected subcutaneously (SC). Statistical difference with PBS-treated mice is indicated: **: p ≤ 0.01 ***: p ≤ 0.001.

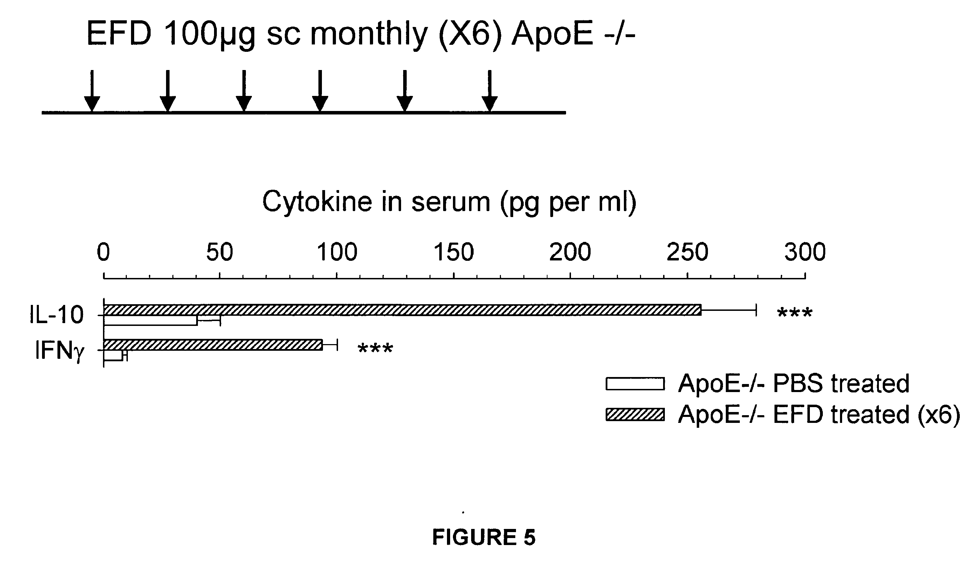

- figure 5 illustrates the cytokines and chemokines increased in the sera of ApoE^{-/-} mice monthly treated 6 times with 100 µg of EFD injected subcutaneously as compared to PBS-treated mice.

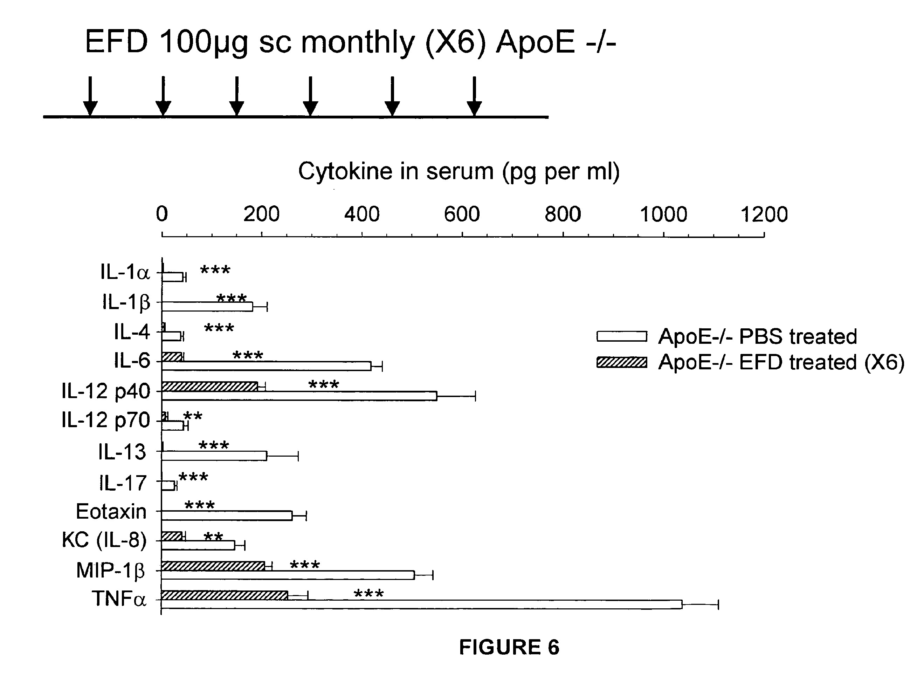

- figure 6 illustrates cytokines and chemokines reduced in the sera of ApoE^{-/-} mice monthly treated 6 times with 100 µg of EFD injected subcutaneously as compared to PBS-treated mice.

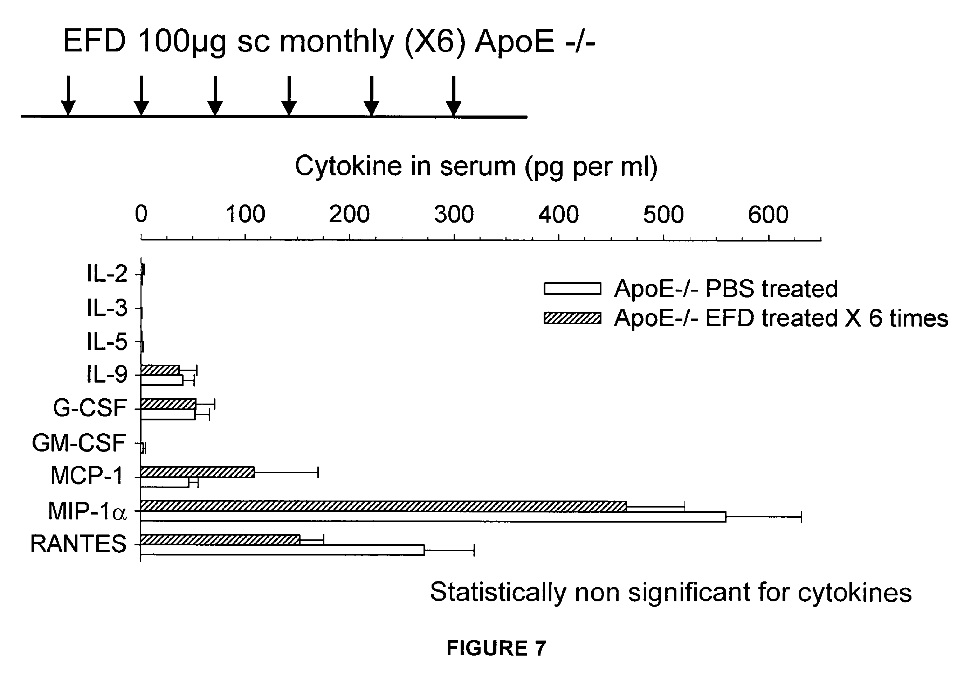

- figure 7 illustrates cytokines remaining unchanged in the sera of ApoE^{-/-} mice monthly treated 6 times with 100 µg of EFD injected subcutaneously as compared to PBS-treated mice.

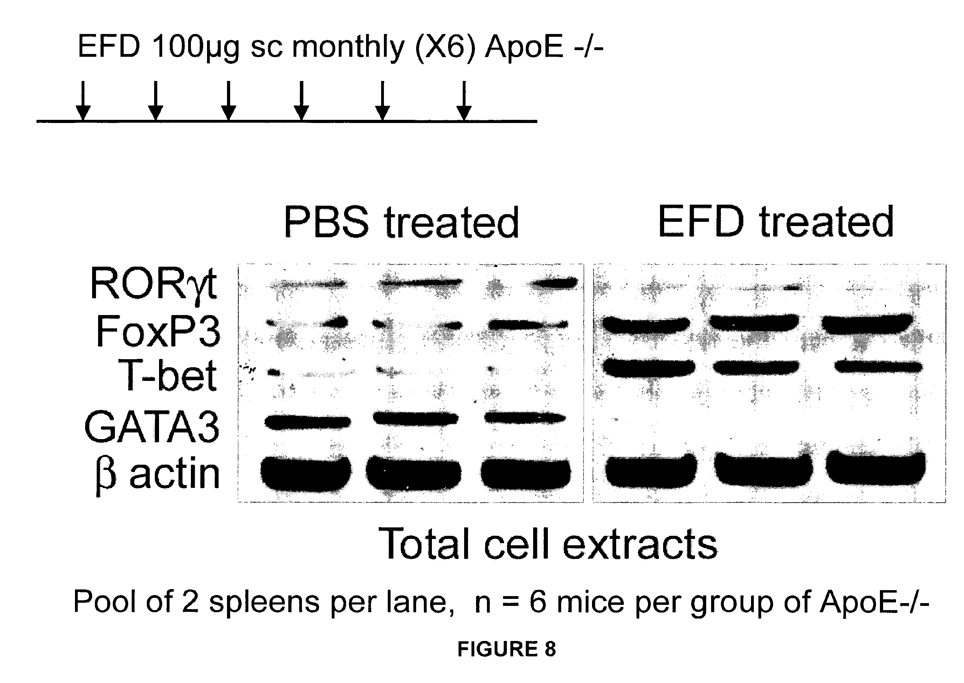

- figure 8 illustrates transcription factors expression in protein extracts of spleen cells of ApoE^{-/-} mice monthly treated 6 times or not with EFD injected subcutaneously. RORγt (Th17signature) and GATA-3 (Th2 signature) were decreased after EFD treatment, whereas, the expressions of FOXP3 (Treg signature) and T-bet (Th1 signature) were enhanced.

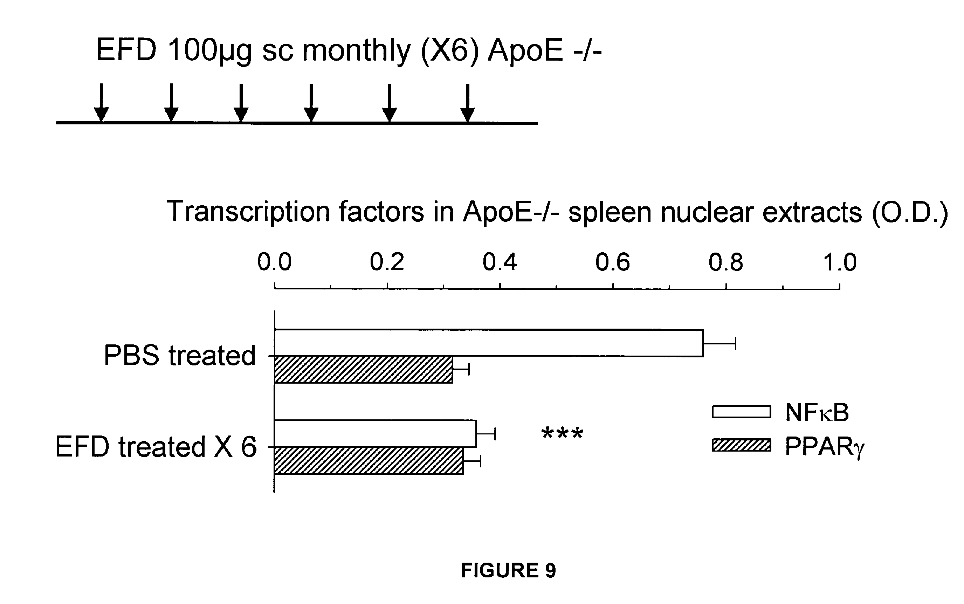

- figure 9 illustrates the activation of NFκBp65 and the expression of PPARγ in spleen nuclear extracts of ApoE^{-/-} mice monthly treated 6 times with EFD injected subcutaneously. EFD treatment significantly reduced NFκB activation and enhanced PPARγ expression. Statistical difference with PBS-treated mice is indicated: ***: p ≤ 0.001.

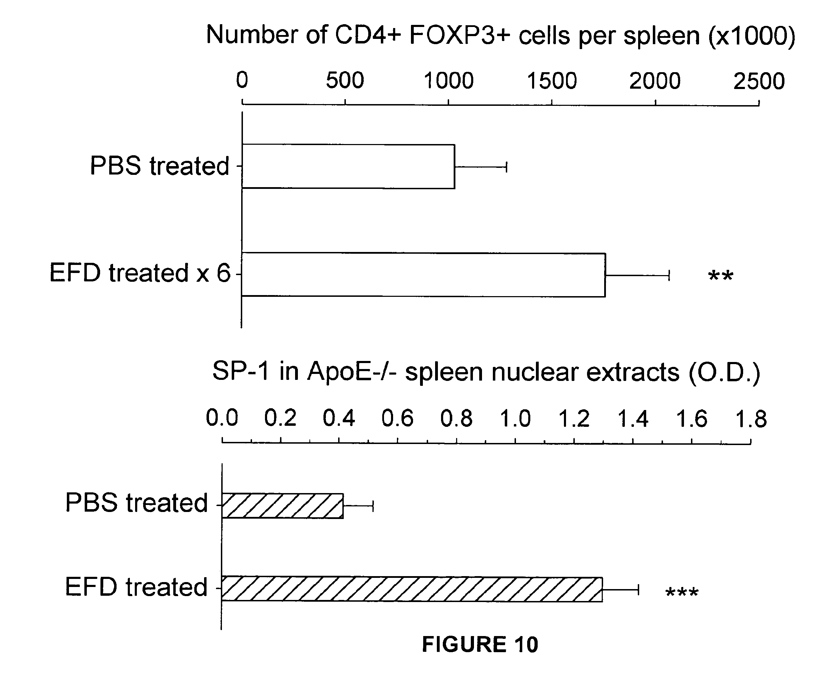

- figure 10 illustrates the numbers of CD4⁺FOXP3⁺ T cells (Treg cells) in the spleen of ApoE^{-/-}mice, those monthly treated 6 times with EFD have a significantly higher number of Tregs than PBS treated mice. The higher expression of the SP-1 transcription factor (associated with IL-10) correlates with the higher production of IL-10 found in the sera of EFD treated mice (Figure 5). Statistical difference with PBS-treated mice is indicated: **: p ≤ 0.01 ***: p ≤ 0.001.

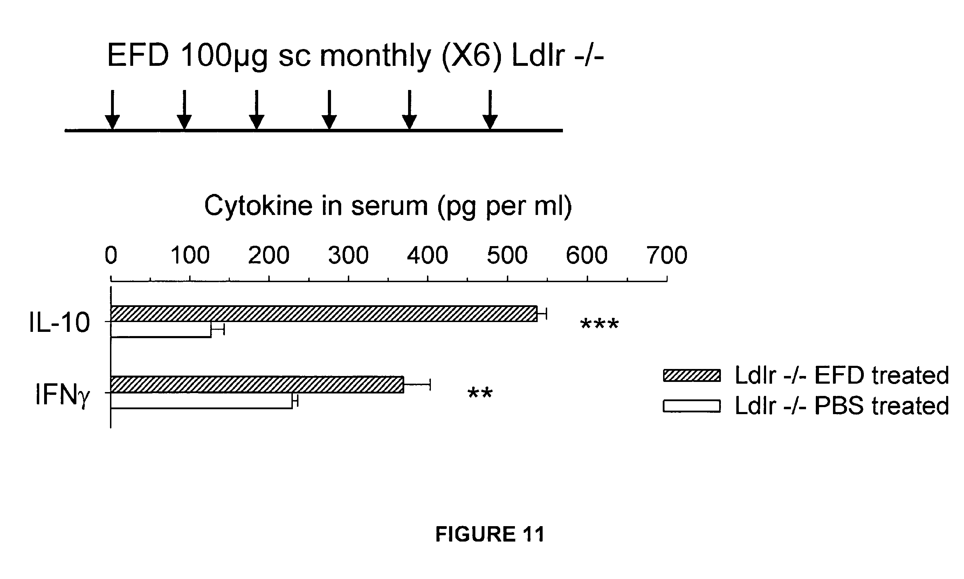

- figure 11 illustrates cytokines increased in the sera of Ldlr-/- mice monthly treated 6 times with 100 µg of EFD injected subcutaneously as compared to PBS-treated mice. Statistical difference with PBS-treated mice is indicated: **: p ≤ 0.01 ***: p ≤ 0.001.

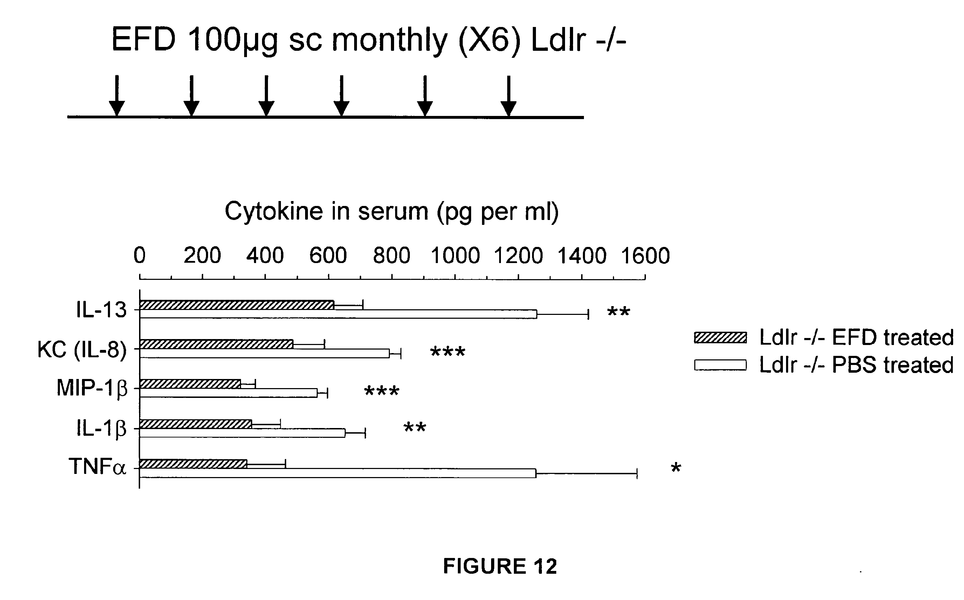

- figure 12 illustrates cytokines and chemokines reduced in the sera of Ldlr-/- mice monthly treated 6 times with 100 µg of EFD injected subcutaneously as compared to PBS-treated mice. Statistical difference with PBS-treated mice is indicated: * :p ≤ 0.05 **:p ≤ 0.01 ***: p ≤ 0.001.

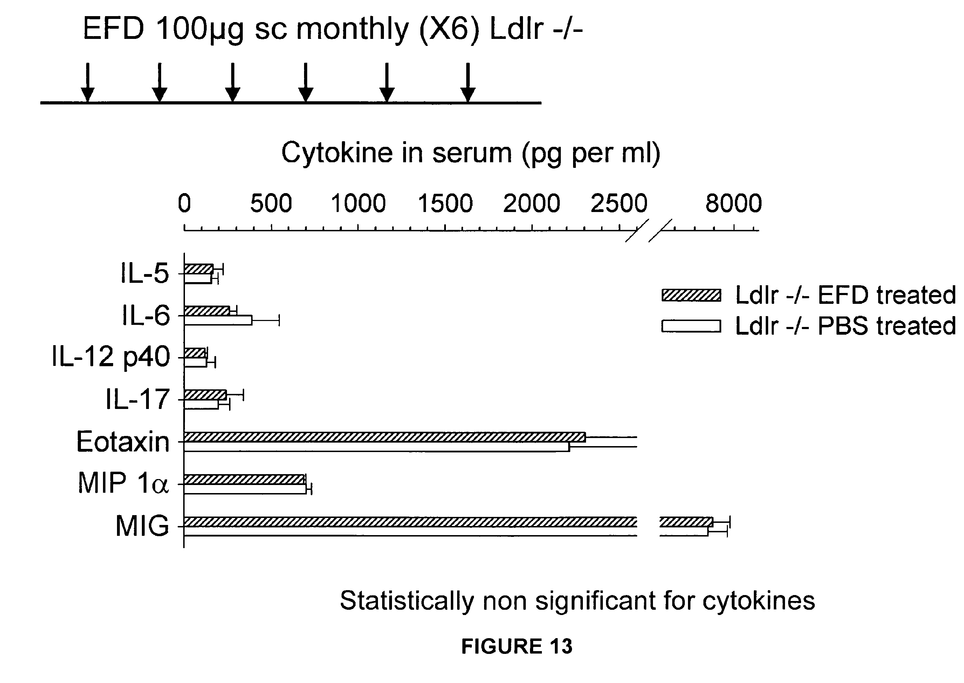

- figure 13 illustrates the cytokines and chemokines remaining unchanged in the sera of Ldlr-/- mice monthly treated 6 times with 100 µg of EFD injected subcutaneously as compared to PBS-treated mice.

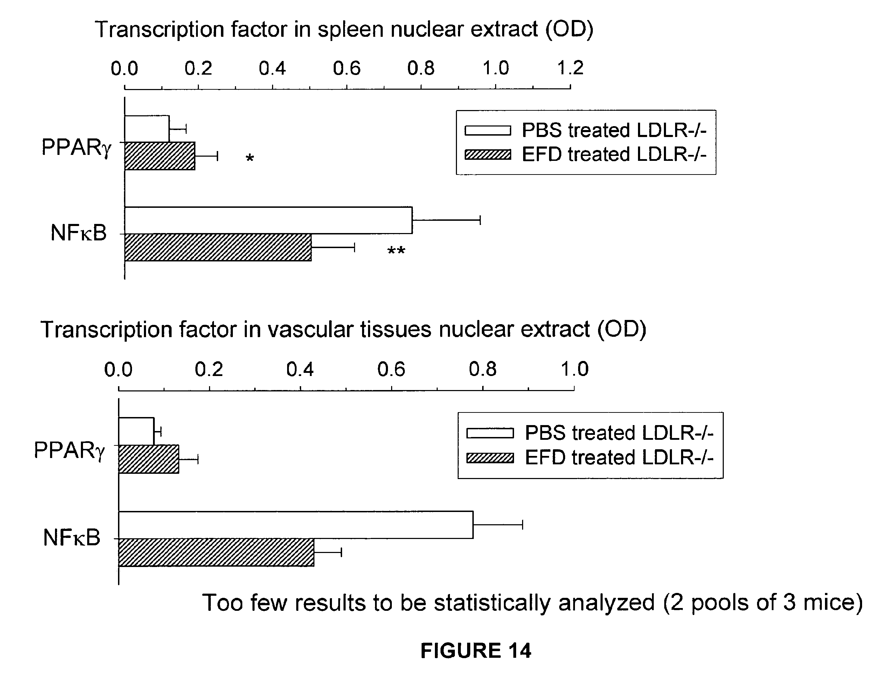

- figure 14 illustrates the NFκB activation and PPARγ expression in nuclear spleen and vascular tree tissues extracts in Ldlr^{-/-} mice monthly treated 6 times or not with 100 µg of EFD injected subcutaneously. NFκB was decreased and PPARγ increased in both organs. Statistical difference with PBS-treated mice is indicated: *: p ≤ 0.05 **: p ≤ 0.01.

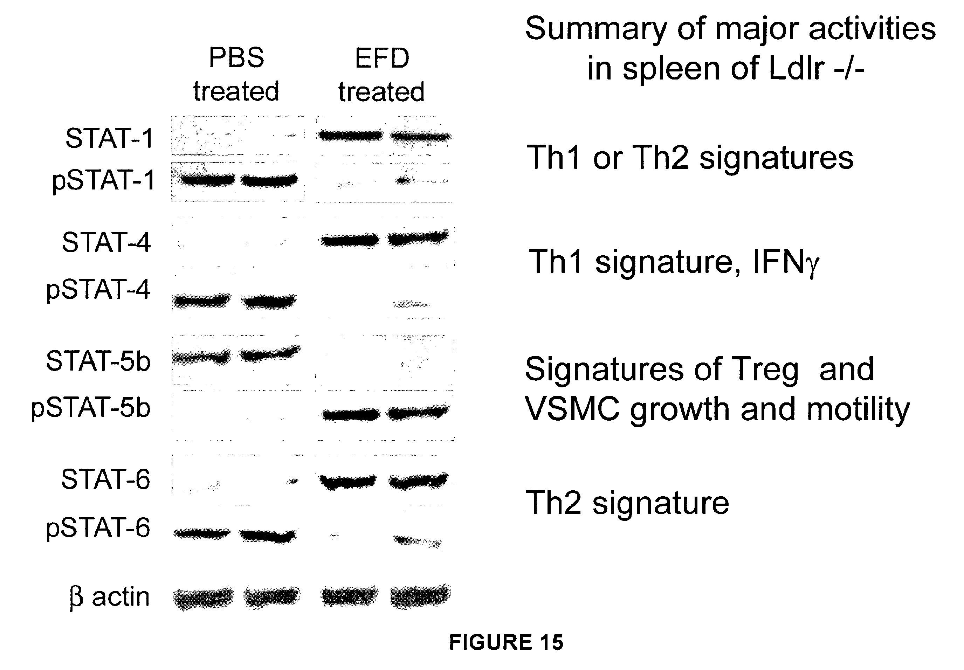

- figure 15 illustrates transcription factors signaling and activating transcription (STAT), in protein extracts of spleen cells of Ldlr^{-/-} mice monthly treated 6 times or not with 100 µg of EFD injected subcutaneously. The phosphorylation of the STATs traduced their activation. EFD induced the phosphorylation of STAT-5b signature of Tregs and VSMC growth and motility, whereas, STAT-1, STAT-4, and STAT-6, signatures of Th1 and Th2 inflammatory cytokines, were phosphorylated only in PBS-treated mice.

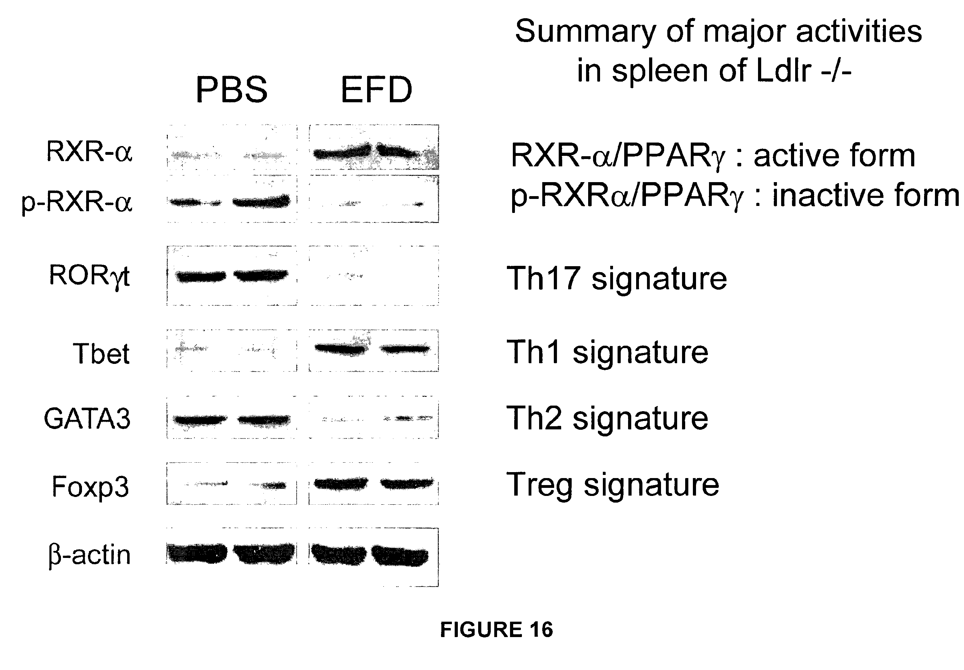

- figure 16 illustrates transcription factors present in the protein extracts spleen cells of Ldlr^{-/-} mice monthly treated 6 times or not with 100 µg of EFD injected subcutaneously. The active form of RXRα is expressed only in EFD-treated mice whereas the phophorylated inactive form is expressed in non-treated mice. RORγt, GATA-3, respectively Th17 and Th2 signatures were decreased, whereas T-bet (Th1 signature) and FOXP3 (Treg signature) were increased after EFD treatment.

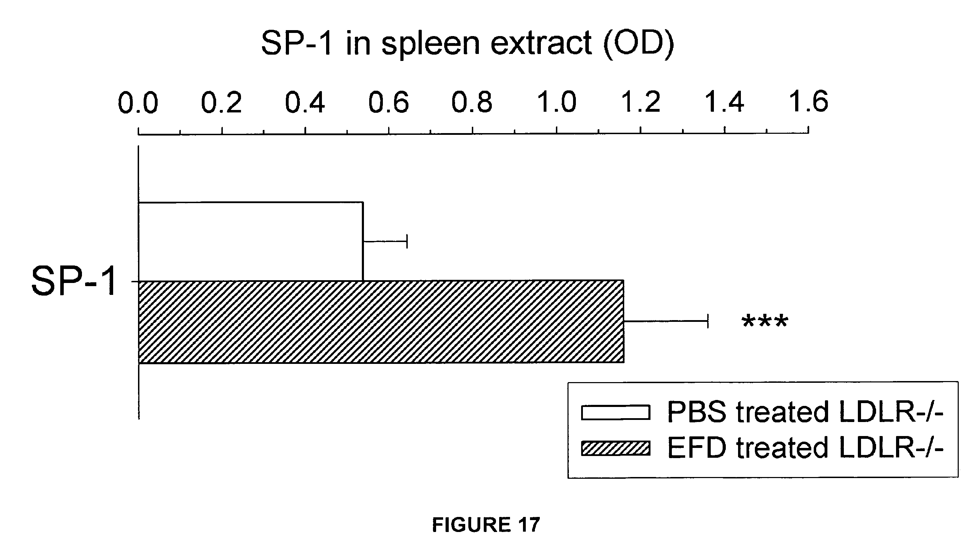

- figure 17 illustrates the expression in protein extracts of spleen cell of Ldlr^{-/-} mice monthly treated 6 times or not with 100 µg of EFD injected subcutaneously, of SP-1, a transcription factor signature of IL-10 production. SP-1 is very significantly enhanced after EFD treatment. Statistical difference with PBS-treated mice is indicated: ***: p ≤ 0.001.

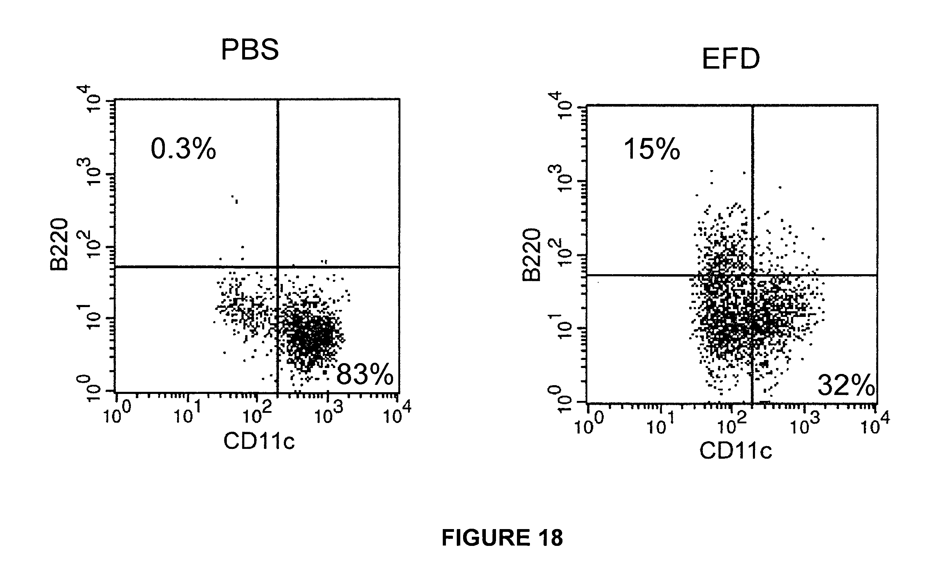

- figure 18: EFD treatment induced the recruitment of plasmacytoid dendritic cells (pDCs). Four days after subcutaneous injection of 100 µg of EFD, pDCs (CD11clow B220high) were more numerous in the draining lymph nodes of Ldlr^{-/-} mice than after PBS injection (15% versus 0.3%) . By contrast the number of cDCs (CD11chigh B220neg) was higher after PBS than EFD (83% versus 32%).

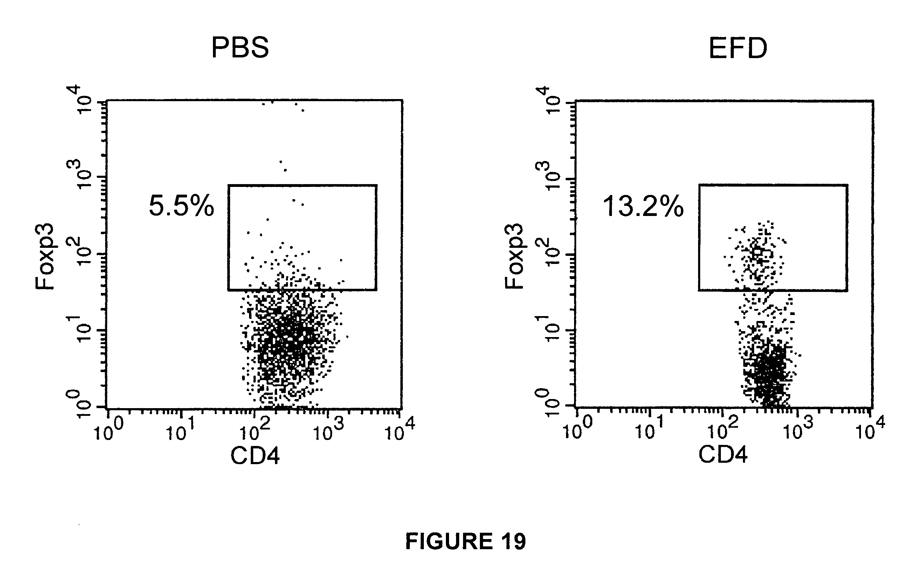

- figure 19: Plasmacytoid dendritic cells (pDCs) promoted regulatory T cells (Tregs). Four days after subcutaneous injection of 100 µg of EFD, the number of Tregs (CD4+Foxp3+) was higher in the draining lymph nodes of Ldlr^{-/-}mice than after PBS injection (13.2% versus 5.5%).

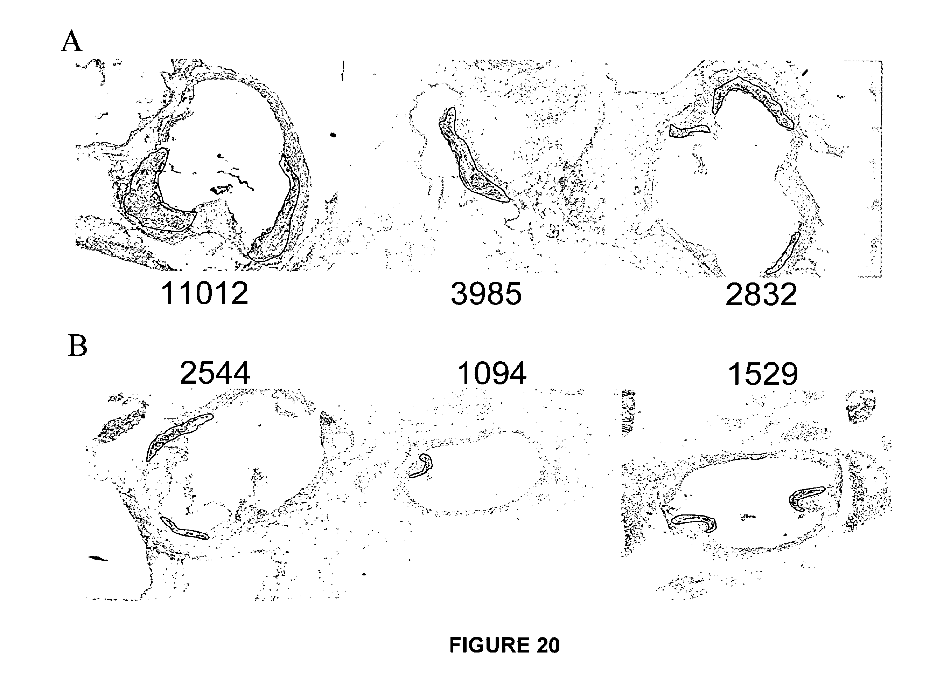

- figure 20 illustrates the atherosclerotic plaques in the aortic sinus of three ApoE^{-/-} mice monthly treated 6 times with PBS (A) or EFD (B). The MOMA-2⁺ macrophages labeled in black are less numerous and the area of the plaques is less extended in EFD-treated mice.

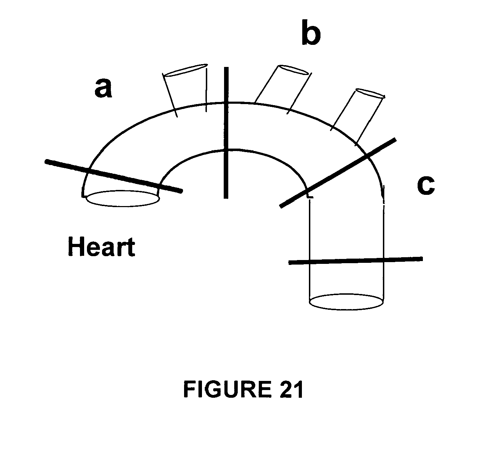

- figure 21 represents the aortic arch of mice EFD-treated or not and the different sites (a, b, c) where the area of the atheroma plaques was semi-quantified on hematoxylin-eosin slides.

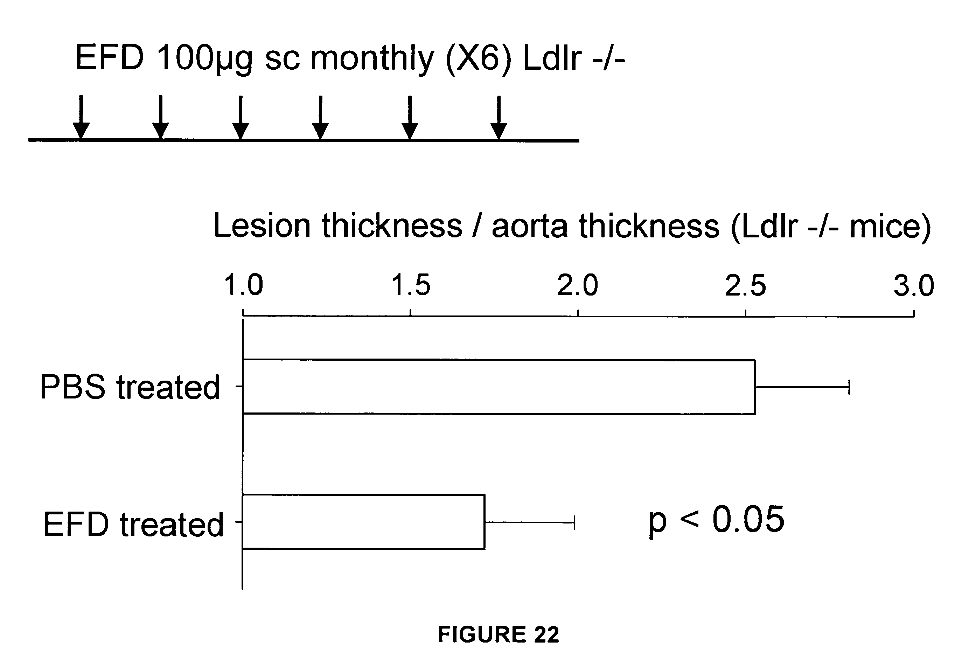

- figure 22: Ldlr-/- mice fed with atherosclerotic diet received (subcutaneously) 100 µl of PBS or 100 µg of EFD at 6, 10, 14, 18, 22 and 26 week-old. Mice were killed at 30 week-old and the mean lesion thickness and aortic thickness were calculated from eight to sixteen aorta arch sections of each mouse. Lesion thickness was analyzed using Leica QWin image analysis software. Results are expressed as the ratio lesion thickness/aorta thickness.

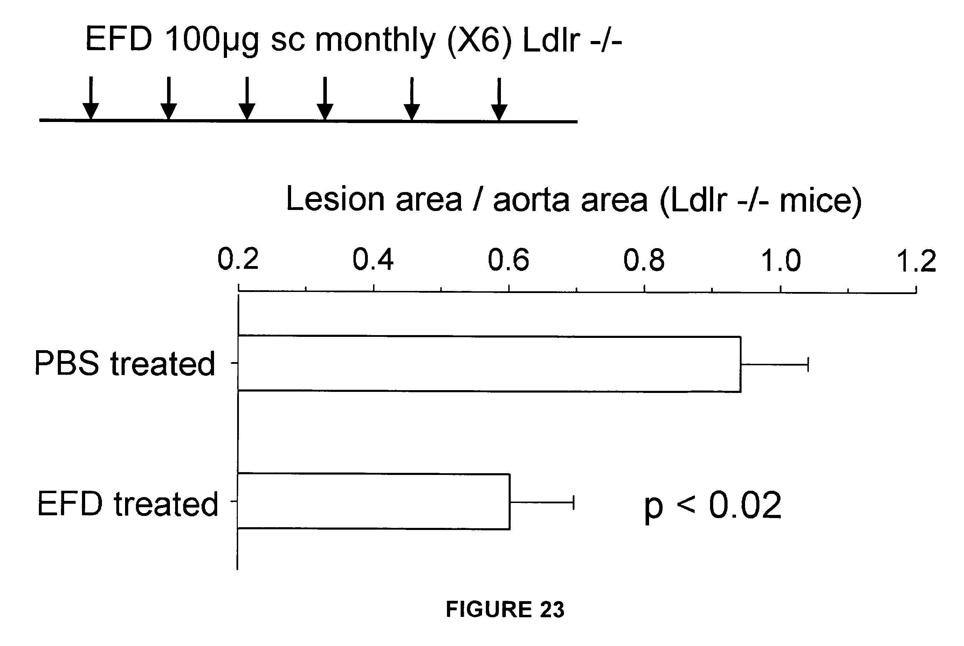

- figure 23: Ldlr-/- mice fed with atherosclerotic diet received (subcutaneously) 100 µl of PBS or 100 µg of EFD at 6, 10, 14, 18, 22 and 26 week-old. Mice were killed at 30 week-old and the mean lesion area and total aortic area were calculated from eight to sixteen aorta arch sections of each mouse. Lesion aera was analyzed using Leica QWin image analysis software. Results are expressed as the ratio lesion aera /aorta aera.

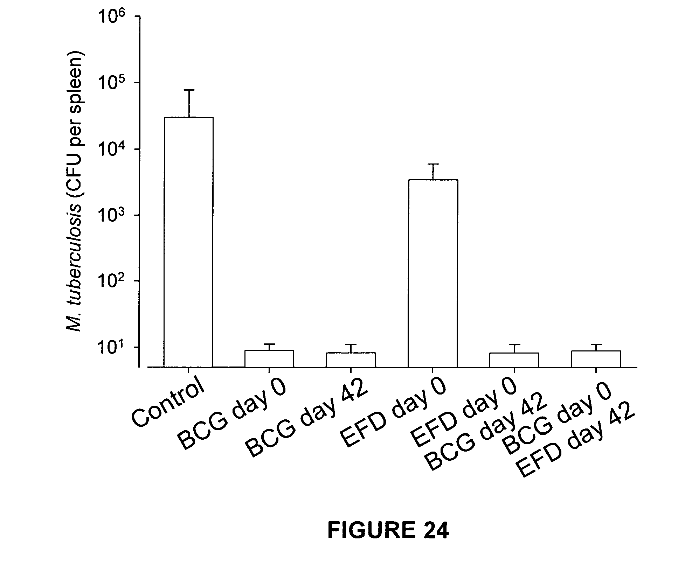

- figure 24: EFD treatment does not interfere with *M tuberculosis* infection or BCG vaccination. Guinea-pigs were treated with EFD before or after BCG injection or without BCG injection. Then, the guinea-pigs were infected with *M. tuberculosis* and the number of living *M tuberculosis* bacteria in the spleen was counted.

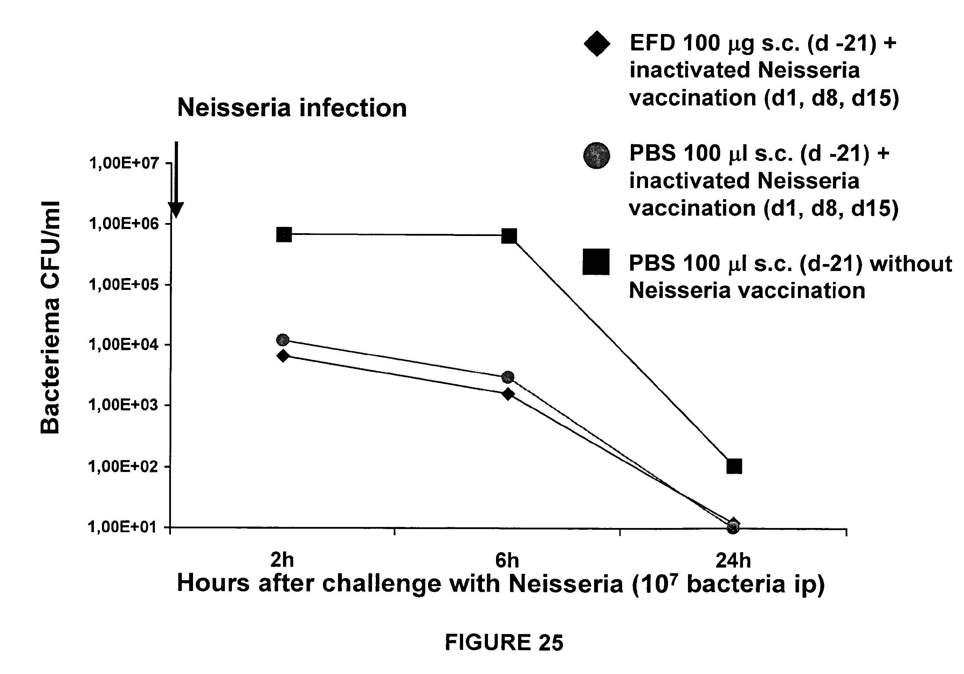

- figure 25: EFD treatment does not interfere with *Neisseria meningitidis* vaccination. BALB/C mice were treated with EFD before *Neisseria meningitidis* vaccination. Two weeks after the last vaccine injection, vaccinated and non-vaccinated mice were challenged with 10⁷ CFU of virulent Neisseria meningitides (ip) and the bacteremia was measured 2, 6 and 24 hours after the challenge. (◆) EFD 100 µg s.c. (d -21) + inactivated Neisseria vaccination (d1, d8, d15). (•) PBS 100 µl s.c. (d -21) + inactivated Neisseria vaccination (d1, d8, d15). (■) PBS 100 µl s.c. (d-21) without Neisseria vaccination.

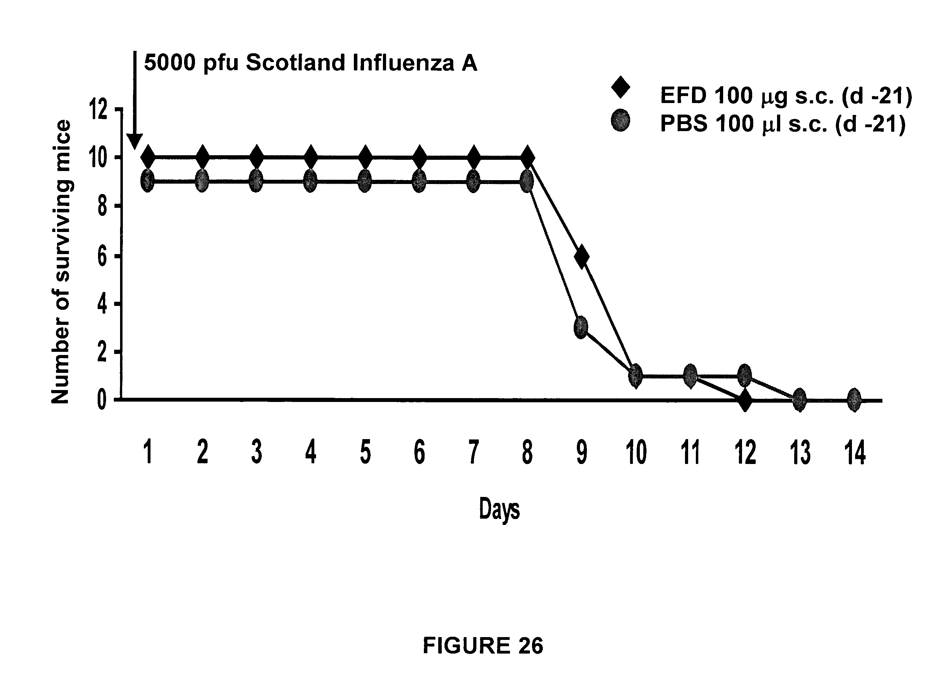

- figure 26: EFD treatment does not modify the course of influenza virus infection. (◆) BALB/C mice were treated with EFD 100 µg subcutaneously (s.c.) (d -21). (•) BALB/C mice were treated with PBS 100 µl subcutaneously (s.c.) (d -21). Twenty-one days later, both groups were intranasally infected with Influenza virus Scotland A strain (5000 pfu), the clinical symptoms and the survival were daily recorded until day 14.

### EXAMPLE 1: Preparation of EFD

*Mycobacterium bovis* BCG Pasteur strain (1173P2) killed by extended freeze-drying (EFD) was prepared as described previously in the International PCT Application WO 03/049752. The EFD preparation contained less than 1.5 % water at the end of the procedure, as determined with a coulometer by using the Karl-Fischer method (METROHM). Ten milligrams of EFD was cultured on Middlebrook 7H 10 agar plates to confirm the absence of living bacteria.

The EFD was resuspended in mannitol (5 %) at a final concentration of 1 mg/mL, freeze-dried for 72 hours under 0.040 mBar pressure (at this step the aim of the freeze-drying is only to obtain a dry composition) and finally resuspended in distillated water (final concentration of 1 mg/ml) before to be injected to mice.

### EXAMPLE 2: Evaluation of EFD treatment on the prevention of atherosclerosis in ApoE^{-/-} and Ldlr^{-/-} C57Bl/6 mice models

### 1) Material and methods

### a) Experimental models of atherosclerosis induction/prevention

Apolipoprotein E deficient (ApoE^{-/-}) and low-density lipoprotein receptor-deficient (Ldlr^{-/-}) male C57B1/6 mice were bred at the animal care facilities of the INRA campus at Jouy en Josas. Groups of 6-7 mice (5-6 weeks old) at the beginning of the experiment were distributed:
1) ApoE^{-/-} mice from group 1 received three times 100 µl of PBS subcutaneously every 10 weeks (bimonthly) and were sacrificed 2 months after the last injection (34 weeks old).
2) ApoE^{-/-} mice from group 2 received 100 µg of EFD subcutaneously at the same intervals than group 1 and were sacrificed 2 months after the third injection of EFD (34 weeks old).
3) ApoE^{-/-} mice from group 3 received 100 µl of PBS subcutaneously every 4 weeks (6 injections) and were sacrificed 1 month after the last injection (30 weeks old) (30 weeks old).
4) ApoE^{-/-} mice from group 4 received 100 µg of EFD subcutaneously at the same intervals than group 3 and were sacrificed 1 month after the sixth injection.
5) Ldlr^{-/-} mice from group 5 received 100 µl of PBS subcutaneously every 4 weeks (6 injections) and were sacrificed 1 month after the last injection (30 weeks old).
6) Ldlr^{-/-} mice from group 6 received 100 µg of EFD subcutaneously at the same intervals than group 5 and were sacrificed 1 month after the sixth injection (30 weeks old).
ApoE^{-/-} mice were fed with conventional food whereas Ldlr^{-/-} mice received food supplemented with lipids and cholesterol (Genestil), from the first injection of EFD (5-6 weeks old) until the end of the experiment.

### b) FACS analysis

A half spleen was crushed on a cell strainer to extract the cells that were labeled firstly with an anti-CD4 antibody, and then, after permeabilization, with an anti FOXP3 antibody (Kit EBIOSCIENCES) according to the manufacturer's recommendations. A FACScan (BD) was used for analytical flow cytometry of the cells.

### c) Transcription factor assay

The other halves of the spleens (poll of 2 mice) were frozen at -25°C to be stored during some days. The proteins were extracted and resolved on 7.5 % SDS-PAGE. Protein bands transferred to nitrocellulose sheets were probed with mouse monoclonal anti-FOXP3, -T-bet, -GATA-3, -RORγt, RXRα, p-RXRα, STAT-1, pSTAT-1, STAT-4, p-STAT-4, STAT-5b, p-STAT-5b, STAT-6, p-STAT-6 (SANTA CRUZ BIOTECHNOLOGY) or β-actin mouse monoclonal antibody (Ac-15, ABCAM). Polyclonal goat anti-rabbit (DAKO CYTOMATION) or goat anti-rabbit IgG (SANTA CRUZ BIOTECHNOLOGY), both HRP- conjugated, were used as secondary antibodies. The immune complex was visualized with an enhanced chemiluminescence detection system (AMERSHAM).

Nuclear proteins were extracted from spleen or vascular tree homogenates (pool of 3 mice) after protease inhibitor treatment. Nuclear extracts (10 µg) were tested for NFκB activation or PPARγ expression with NFκBp65 or PPARγ TransAM^{™} transcription factor assay kits (ACTIVE MOTIF) according to the manufacturer's recommendations. The SP1 expression in spleen nuclear extracts was also tested by TransAM^{™} transcription factor assay kit (ACTIVE MOTIF).

### d) Cytokine assays

Pro-inflammatory cytokines and chemokines (IL-1α, IL-1β, IL-6, IL-13, IL-17, KC (IL-8), IL-12p40, IL-12p70, IFNγ, TNFα, MIP-1β, Eotaxin) and anti-inflammatory (IL-10) cytokines were measured in the sera. The Bio-Plex cytokine assay (BIO-RAD) was used to measure the cytokine and chemokine contents.

### e) Immunohistochemical studies

Sinus and aortas were collected on EFD-treated and PBS-treated ApoE-/-mice and Ldlr^{-/-} mice were frozen in Optimum Cutting Temperature (OCT; SAKURA FINETEK). The frozen blocks were kept at -80°C until cryostat sectioning. Aortic sinus sections (5µ) of ApoE-/-mice were cut in a cryostat kept at -20°C and collected on glass slides. Consecutive sections of each block were stained with hematoxylin-eosin and anti-MOMA-2 to visualize macrophages in the atherosclerotic plaques. Aorta-arches of Ldlr^{-/-} mice were cryosectioned. Sections were air-dried and fixed with ice-cold acetone and then hematoxylin-Eosin stained. Lesion area and thickness in three different sites (a, b, c) of the aortic arch was analyzed using Leica QWin image analysis software. The results were expressed as a score (lesion area) for each site. In addition, the mean lesion thickness and surface and mean aortic thickness and surface were calculated from eight to sixteen aorta arch sections of each mouse Results were expressed as the ratio lesion thickness/aorta thickness and lesion area/aorta area.

Aortas were collected from EFD-treated and PBS-treated Ldlr^{-/-} mice, fixed in 4 % formaldehyde, opened longitudinally and stained with Sudan IV. Lipid lesions appeared in red.

### 2) Results

### a) Cytokines and chemokines in the sera

In ApoE^{-/-} mice receiving either 3 or 6 injections of EFD (groups 2 and 4), IL-10 (a cytokine known to be anti-inflammatory) and IFN-γ were enhanced (figures 1 and 5), inflammatory cytokines and chemokines (IL-1α, IL-1β, IL-4, IL-6, IL-12p40, IL-12p70, IL-13, IL-17, Eotaxin, KC (IL-8), MIP-1β and TNF-α) were similarly significantly reduced as compared to PBS treated mice (figures 2 and 6). The other cytokines and chemokines tested (IL-2, IL-3, IL-5, IL-9, G-CSF, GM-CSF, MCP-1, MIP-1α and RANTES) remained unchanged after EFD treatment (figures 3 and 7).

In Ldlr^{-/-} mice receiving 6 injections of EFD (group 6) IL-10 and IFN-γ were enhanced similarly to what was observed for ApoE^{-/-} (figure 11). Among the "pro-inflammatory" cytokines and chemokines tested, IL1-β, MIP1-β, IL-13, KC (IL-8) and TNF-α were significantly reduced as compared to non-treated mice (figure 12). It has to be noted that EFD treatment failed to reduce IL-6, IL-12p40, IL-17, and Eotaxin in Ldlr-/- mice whereas these cytokines and chemokines were significantly reduced in ApoE^{-/-} mice after 3 or 6 EFD treatments. Other cytokines and chemokines remained unchanged (IL-5, MIP-1α, MIG) (figure 13).

### b) Transcription factors in nuclear and in total spleen cells extracts

In spleen cells nuclear extracts of ApoE^{-/-} mice receiving 3 or 6 injections of EFD, NFκBp65 was significantly decreased whereas PPARγ expression was enhanced or stabilized (figures 4 and 9)

In total protein extracts from spleen cells, after 6 EFD treatments, the expression of RORγt (signature of inflammatory Th17 cells) was slightly reduced and GATA-3 (signature of Th2 cells) inhibited. The expression of T-bet (signature of Th1 cells) and FOXP3 (Treg cells) were strongly enhanced (figure 8). The SP-1 transcription factor associated to IL-10 production was significantly enhanced (figure 10) in correlation with high levels of IL-10 found in the sera of EFD-treated mice. FACS analysis of spleen cells showed a significantly higher number of CD4+FOXP3+ Treg cells in the EFD-treated mice than in the PBS-treated mice (6 injections) (figure 10).

In Ldlr^{-/-} mice, 6 injections of EFD reduced significantly NFκB activation and enhanced PPARγ expression in nuclear spleen extracts from 6 mice (figure 14). Similar results were obtained from two extracts of vascular tree tissues of pool of 3 mice (the number of pools was too low to permit a statistical analysis) (figure 14). The tendency of reducing NFκB and enhancing PPARγ must be confirmed by more vascular tissue pools. Signaling and activating factors were also measured in protein extracts from spleen cells. After EFD treatment, phosphorylation of STAT-5b, a transcription factor reported to activate Treg response and vascular smooth muscle cells growth (VSMC) was observed. By contrast, STAT-1, STAT-4, STAT-6, signatures of Th1 and Th2 inflammatory responses, were phosphorylated only in PBS treated mice (figure 15).

To activate transcription, PPARγ requires heterodimerization with the retinoid X receptor α (RXRα) while phosphorylated-RXRα and p-RXRα-PPARγ complex are inactive. In correlation with enhanced PPARγ expression in nuclear spleen cells extracts of EFD treated mice, RXRα expression is enhanced after EFD treatment, associated with a blockage of its phosphorylation, while p-RXRα inactive form was expressed in PBS treated mice (figure 16).

In Ldlr^{-/-} mice, EFD treatment reduced RORγt and GATA-3 expressions whereas T-bet and FOXP3 and SP-1 expressions were enhanced (figure 16), as found also in ApoE^{-/-} mice (figure 8). A higher expression of SP-1, transcription factor correlating with IL-10 production, was observed after EFD treatment of Ldlr^{-/-} mice (figure 17) as reported previously for ApoE^{-/-}mice (figure 10).

### c) Plasmocytoid dendritic cells (pDCs) and regulator T cells (regs)

EFD treatment induced the recruitment of plasmacytoid dendritic cells (pDCs). Four days after subcutaneous injection of 100 µg of EFD, pDCs (CD11clow B220high) were more numerous in the draining lymph nodes of Ldlr^{-/-} mice than after PBS injection (15% versus 0.3%; figure 18) . By contrast the number of cDCs (CD11chigh B220neg) was higher after PBS than EFD (83% versus 32%).

Plasmacytoid dendritic cells (pDCs) promoted regulatory T cells (Tregs). Four days after subcutaneous injection of 100 µg of EFD, the number of Tregs (CD4+Foxp3+) was higher in the draining lymph nodes of Ldl^{-/-}mice than after PBS injection (13.2% versus 5.5%; figure 19).

### d) Atherosclerotic plaques

MOMA-2⁺ macrophages in atherosclerotic plaques were less numerous in ApoE^{-/-} mice receiving 6 EFD injections than in PBS-treated mice controls (figure 20). The plaques of EFD-treated mice were also less extended than those of PBS-treated mice (figure 20). This was confirmed by a semi-quantification of the area of the atheroma plaques in three different sites (a, b or c) of the aortic arch (figure 21), for each Ldlr^{-/-} mice of the PBS-treated group (6 mice) and each Ldlr^{-/-} mice mice of the EFD-treated group (6 mice). The mean value at each site is also indicated for the PBS-treated and EFD-treated groups (Table I).

**Table I: Quantification of atheroma plaques in different sites of the aortic arch**

| **PBS- treated mice** | **Lesion area (arbitrary units) in different sites of aortic arch** | | | **EFD- treated mice** | **Lesion area (arbitrary units) in different sites of aortic arch** | | |
|---|---|---|---|---|---|---|---|
| | **a** | **b** | **c** | | **a** | **b** | **c** |
| **N°1** | 3 | 3 | ND | **N°1** | 2 | - | ND |
| **N°2** | 0.5 | ND | 2 | **N°2** | 2 | 3 | 3 |
| **N°3** | 4 | 4 | 1 | **N°3** | 2 | 2 | 1 |
| **N°4** | 3 | 4 | 4 | **N°4** | 3 | 2 | 1 |
| **N°5** | ND | 4 | 4 | **N°5** | 3 | 2 | 1 |
| **N°6** | 4 | 3 | 3 | **N°6** | 2 | 1 | ND |
| **mean** | 2.9 | 3.6 | 2.8 | **mean** | 2.4 | 1.6 | 1.5 |

| | | | | | | | |
|---|---|---|---|---|---|---|---|
| * ND: non-determined | | | | | | | |

In the EFD treated mice, the lesions were statistically smaller in site b (p = 0.015; Mann-Whitney test).

The quantification of the lesion area and thickness in the 6 EFD-treated and PBS-treated Ldlr^{-/-} mice (figures 22 and 23) demonstrated that the lesion area and thickness were significantly reduced in the EFD-treated Ldlr^{-/-} mice. The Sudan IV staining of EFD-treated and PBS-treated Ldlr^{-/-} mice aortas fixed and opened longitudinally indicated that the lipid lesions were drastically reduced in the EFD-treated Ldlr^{-/-} mice.

### EXAMPLE 3: EFD treatment does not interfere with M.tuberculosis, Leishmania major or influenza virus infections or impair BCG or Neisseria vaccination

### a) M. tuberculosis infection and BCG vaccination

Guinea-pigs, a species more susceptible to *M. tuberculosis* than mice, were injected with:
- Group 1: PBS (100µl) on day 0 (control)
- Group 2: BCG (10⁶ colony forming units (CFU) in 100µl PBS) on day 0
- Group 3: BCG (10⁶ colony forming units (CFU) in 100µl PBS) on day 42
- Group 4: EFD ((100µg) in 100µl of PBS) on day 0
- Group 5: EFD ((100µg) in 100µl of PBS) on day 0 and BCG (10⁶ colony forming units (CFU) in 100µl PBS) on day 42
- Group 6: BCG (10⁶ colony forming units (CFU) in 100µl PBS) on day 0 and EFD ((100µg) in 100µl of PBS) on day 42.

On day 80, all the groups were challenged with *Mycobacterium tuberculosis* (H37Rv; 5.10⁵ CFU). The guinea pigs were sacrificed at day 122. The spleens were collected and homogenized. Appropriate dilutions fo homogenized spleens were plated on Middlebrook 7H10 agar medium (DIFCO). The plates were incubated at 37°C for one month and the number of colony forming units (CFU) of M. tuberculosis was counted.

EFD treatment, before or after BCG vaccination or without BCG vaccination, did not sensitize to *M tuberculosis* infection or impair BCG vaccination (Figure 24).

### b) Leishmania major infection assay

BALB/c mice (non healer mice) and C57B1/6 mice (healer mice), 8 per group, were injected (Day 0) at the base of the tail with: 100µl of PBS or EFD (100 µg) in 100µl of PBS. On day 45, they received 10 to 20 viable *Leishmania major* parasites in 10µl, in right ear.

Local inflammations were observed and measured during 6 months: no differences were observed between PBS and EFD treated groups.

Metastatic lesions were recorded on day 160.

In the PBS-treated group, all mice (except one) had small to medium local lesions: 5 had normal tail, 1 with small lesion at the base of the tail and 2 had lost their tail and they had lesion on rear footpads.

In the EFD-treated group, all mice had small to medium local lesions: 7 had their tail, 2 with a small lesion at the base of the tail and 1 had lost its tail, with lesions on both rear footpads. No differences (similar lesions) were observed between PBS and EFD treated groups of BALB/c mice (non healer mice). No differences (absence of lesion on all mice) were observed between PBS and EFD injected C57B1/6 mice (healer mice).

These results which show that EFD treatment does not sensitize the mice to *Leishmania major* infection indicate that EFD does not interfere with pathogen infection.

### c) Neisseria vaccination

One group of BALB/c mice was treated subcutaneously with 100 µg of EFD in 100µl, the two other groups received 100 µl of PBS. Twenty-one days later EFD-treated group and one of the PBS-treated group were vaccinated with heat-killed Nesseiria meningitidis (3 doses at one week interval). Two weeks after the last vaccine injection, vaccinated and non-vaccinated mice were challenged with 10⁷ CFU of virulent Neisseria meningitides (ip) and the bacteremia was measured 2, 6 and 24 hours after the challenge. EFD treatment did not modify the protective effect of the vaccination (figure 25).

### d) Influenza virus infection

One group of BALB/c mice was treated subcutaneously with 100 µg of EFD in 100µl, the other group received 100 µl of PBS. Twenty-one days later, both groups were intranasally infected with Influenza virus Scotland A strain (5000 pfu), the clinical symptoms and the survival were daily recorded. No difference was found between EFD-treated and PBS-treated groups (figure 26), indicating that EFD treatment does not modify the course of influenza virus infection.

## Claims

1. Composition comprising *Mycobacterium bovis* BCG bacteria killed by extended freeze-drying for preventing or treating atherosclerosis.

2. The composition according to claim 1, wherein the said *Mycobacterium bovis* BCG bacteria have proteins whose primary structure ispreserved.

3. The composition according to claim 1 or claim 2, wherein said *Mycobacterium bovis* BCG bacteria consists of killed and non-denatured *Mycobacterium bovis* BCG bacteria, containing less than 1.5 % of water.

4. The composition according to anyone of claims 1 to 3, which comprises from about 10 µg to about 10 mg of *Mycobacterium bovis* BCG bacteria killed by extended freeze-drying.

5. The composition according to claim 4, which comprises from about 100 µg to about 1 mg of *Mycobacterium bovis* BCG bacteria killed by extended freeze-drying.

6. The composition according to claim 5, which comprises a unit dose of 100 µg of *Mycobacterium bovis* BCG bacteria killed by extended freeze-drying, said unit dose being intended for subcutaneous administration every 3 to 6 months.

7. The composition according to anyone of claims 1 to 6, where said bacteria are obtainable by a process comprising:
(i) harvesting a culture of living *Mycobacterium bovis* BCG bacteria cells,
(ii) freezing the *Mycobacterium bovis* BCG bacteria cells in water or in an aqueous solution of a salt,
(iii) killing the frozen *Mycobacterium bovis* BCG bacteria cells by drying them in a lyophiliser, for a time sufficient to remove at least 98.5% of the water, and
(iv) collecting the extended freeze-dried *Mycobacterium bovis* BCG killed bacteria cells.

8. Products containing a composition as defined in anyone of claims 1 to 7 and at least another product selected from the group consisting of anti-inflammatory and immunomodulatory drugs, as a combined preparation for simultaneous, separate or sequential use in the prevention or the treatment of atherosclerosis.
